# EUROPEAN PATENT SPECIFICATION

(11) **EP 2 932 940 B1**
(45) Date of publication and mention of the grant of the patent: **11.08.2021**
(21) Application number: 15275096.4
(22) Date of filing: 30.03.2015
(51) Int. Cl.: A61F 2/07

(54) **MAGNETICALLY EXPANDABLE MEDICAL DEVICE**
MAGNETISCH EXPANDIERBARE MEDIZINISCHE VORRICHTUNG
DISPOSITIF MÉDICAL EXTENSIBLE MAGNÉTIQUEMENT

(30) Priority: 14.04.2014 GB 201406658
(43) Date of publication of application: 21.10.2015
(73) Proprietor: Cook Medical Technologies LLC, Bloomington, IN 47404 (US)
(72) Inventor: Hansen, Palle, 4632 Bjaeverskov (DK); Andersen, Torben, 2630 Taastrup (DK)
(74) Representative: Williams Powell

(56) References cited:
- WO-A2-2014/133829
- US-A1- 2009 287 293

## Description

The present invention relates to a magnetically expandable medical device, preferably an implantable medical device, and in the preferred embodiment to a magnetically expandable graft. The teachings herein can also be used in a soft catheter or sleeve, for instance for an introducer assembly.

Implantable medical devices are known in many forms and for treating many medical conditions. Examples include stents, grafts, filters, occluders, valve replacement prostheses and so on. Such devices are generally introduced into the patient endoluminally through a remote percutaneous entry point. In order to achieve this, the medical device is loaded onto a carrier at a distal end of an introducer assembly with the device being held in a radially compressed configuration. The introducer assembly is fed into the patient's vasculature from the percutaneous entry point until its distal end is located at the treatment site. Once so positioned, the medical device is released from the carrier and expanded until the device engages the vessel wall to be held thereby. The device can be of a type which expands automatically, achieved by use of spring material, shape memory material and so on. Other types of device are plastically deformable and expanded by a separate mechanism, for instance by inflation of a delivery balloon on which the device is held in crimped form.

It is important that in use the medical device applies a constant force against the walls of the vessel in which it is located. This ensures good patency to the vessel wall, that is, a good seal between the device and the wall tissue, in order to stop leakage around the device. The application of constant force also ensures that the device does not migrate or rotate out of alignment over time.

The force produced by the above-mentioned medical devices is a mechanical force, be it by a spring force of the components of the device or by relative mechanical stiffness in the case of plastically deformable devices. This requires the devices to have a certain structural strength and as a result a certain volume of material, resulting in increased device profile and reduced compressibility for delivery purposes. In addition, such devices have a relatively high volume of foreign material which is implanted and left in the patient's body.

Furthermore, the structure of such devices can impart unnatural forces on the vessel wall, the most common being a vessel straightening force acting against the natural curvature of the vessel or a significant pressing force against the sides of the vessel. Such forces can lead to restenosis of the vessel.

Some examples of implantable medical devices are disclosed in US 2011/0257724, US 2006/0212113, US 8,449,604 and US 7,722,668. US 2009/0287293 discloses a magnetically induced radial expansion vascular stent consisting of an expandable body member provided with magnetic elements disposed in a parallel pattern.

WO 2014/133829 is a document according to Art. 54(3) EPC, and discloses an embolic protection device.

The present invention seeks to provide an improved implantable medical device and an introducer assembly therefor. The device may be, but is not limited to, a vascular graft.

According to an aspect of the present invention, there is provided a medical device as specified in claim 1.

For example, the magnetic elements may be disposed such that their south poles face in the same direction as the internal surfaces of the body member, with their north poles facing outwardly. Alternatively, the magnetic elements could be oriented in the opposite direction.

The device is preferably an implantable medical device, such as a prosthesis.

A medical device of such a structure does not need to rely upon the generation of a mechanical force as with conventional medical devices, but instead makes use of constant magnetic repulsion to keep the body member of the device in its expanded state against the vessel wall. As a result of the use of such magnetic forces it is not necessary to generate large opening forces to hold the device in place. Moreover, the device can in practice be much more flexible and able to configure to the shape of the vessel or other organ, as well as accommodating changes in the vessel during normal body function and over time. This enhanced flexibility also makes the device suitable for the cerebral vessels.

The device may be any expandable prosthesis. It may be a graft, stent, filter, occluder, valve replacement prosthesis, for example. The teachings herein could also be used for other medical devices, such as catheters and the like, enabling the provision of a soft catheter for delicate vessels for instance.

In an embodiment, the body member may be formed of woven, knitted, braided or sheet material.

The device may be or include a graft, the graft forming the body member.

In one embodiment, the magnetic elements are disks or rods, which may be generally circular, although could have other shapes such as oval, square or rectangular.

There may be provided at least first and second lines of said magnetic elements along the body portion of the device. Preferably, there may be provided at least two pairs of lines of magnetic elements, the lines in each pair being disposed on opposing internal surfaces of the body member. For example, there may be provided four, six or eight lines of magnetic elements, arranged in opposing pairs.

In one embodiment, the lines of magnetic elements extend parallel to an axial or longitudinal dimension of the body member, although in another embodiment the lines of magnetic elements extend at an angle to the axial dimension of the body member. They may, for example, extend helically or in a zigzag manner along the body member. In some embodiments, the magnetic elements may extend circumferentially around the body member, as a simple annular shape or in any other shape including undulating and zigzag.

In another embodiment, the magnetic elements are strips of magnetic material, which may extend parallel to the axial or longitudinal dimension of the body member or at an angle thereto. The strips of magnetic material may have the same shapes as characteristics as the lines of magnetic elements. Furthermore, the strips may be discontinuous along the length of the body member.

The magnetic elements may be painted on or attached to the body member of the device.

In a preferred embodiment, the magnetic elements are formed of paramagnetic material, which can be magnetised once the elements have been fitted to the body member of the device.

Advantageously, the magnetic elements are formed of biodegradable material, preferably of a material which will degrade at a rate slower than a rate of ingrowth of vessel tissue.

Other features of the apparatus and method disclosed herein will become apparent from the following specific description of preferred embodiments.

Embodiments of the present invention are described below, by way of example only, with reference to the accompanying drawings, in which:
Figure 1 is a schematic view in perspective of an embodiment of graft according to the invention;
Figure 2 is a transverse cross-sectional view of the graft of Figure 1;
Figure 3 is a schematic view in perspective of another embodiment of graft according to the invention;
Figure 4 is a schematic view of the graft of Figure 1 disposed in a vessel, the vessel being shown in cross-section;
Figure 5 is a schematic view of the graft of Figure 1 disposed in a curved vessel, the vessel being shown in cross-section; and
Figure 6 is a schematic view of one of the magnetic elements of the device of Figure 1.

The embodiments described below are directed to a graft of tubular and substantially cylindrical form. The teachings herein are particularly suited to vascular grafts, although are also applicable to other forms of medical device, including devices provided with graft elements or other coverings and also medical devices having no graft element, such as stents and so on.

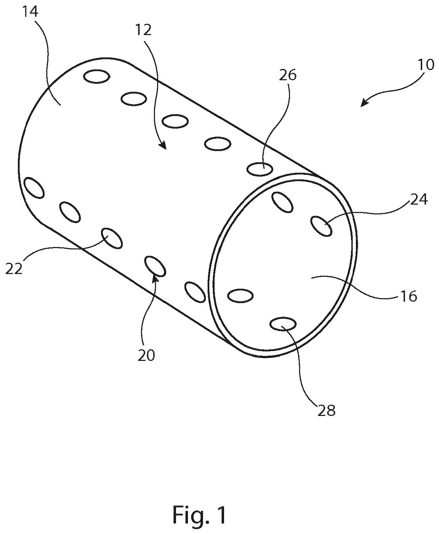

Referring first to Figure 1, the graft 10 shown in schematic form includes a body member 12 of generally tubular form and made of, in this example, a conventional graft material, typically woven or knitted. The graft material could be of ultrahigh molecular weight polyethylene, such as Dyneema™, Expanded polytetrafluoroethylene (ePTFE) or any other suitable or known material. The tubular graft element 12 includes an outer abluminal surface 14 and an inner luminal surface 16. Attached to graft element 12 is a plurality of magnetic elements 20. The elements 20 are, in this example, arranged in four arrays or lines 22, 24, 26 and 28 which extend along the length of the tubular graft element 12 and in this embodiment substantially parallel to the longitudinal axis of the graft element 12. The arrays or lines 22-28 of magnetic elements 20 are arranged in opposing pairs. More specifically, the arrays or lines 22 and 24 are disposed on opposite sides of the internal surface 16 of the graft element 12 and the pair of arrays or lines 26 or 28 are likewise disposed opposite one another. In this example, the four arrays or lines 22, 28 are equally spaced circumferentially around the graft tube 12 so in this example separated from one another by 90° around the graft tube. As is described in further detail below, the magnetic elements 20 each have north and south poles and are disposed on the graft tube such that they have the same pole facing inwardly, towards the central axis of the graft tube. In one embodiment, the south pole of the magnetic elements 20 faces towards the radial centre of the graft tube 12. Thus, all of the magnetic elements 20 have the opposing pole, in this example, the north pole, facing outwardly of the graft tube. Of course, the polarities of all the magnets could be reversed relative to those depicted.

The magnetic elements could in their simplest form be formed of a magnetic material printed or otherwise applied to the material of the graft tube 12 or can be magnetic disks or other three-dimensional structures mechanically affixed to the graft tube 12, for instance by bonding, riveting or the like.

There may be provided at the ends of the graft tube 12 first and second stents for keeping the ends of the graft fully open, thereby ensuring patency with the vessel wall. Suitable stents include stents having a zigzag form for radial compressibility. Each stent could be sewn to a respective end of the graft tube 12 and disposed either inside or outside the graft tube 12. Such stents can be useful in embodiments in which only a few magnetic elements 20 are disposed circumferentially around the graft.

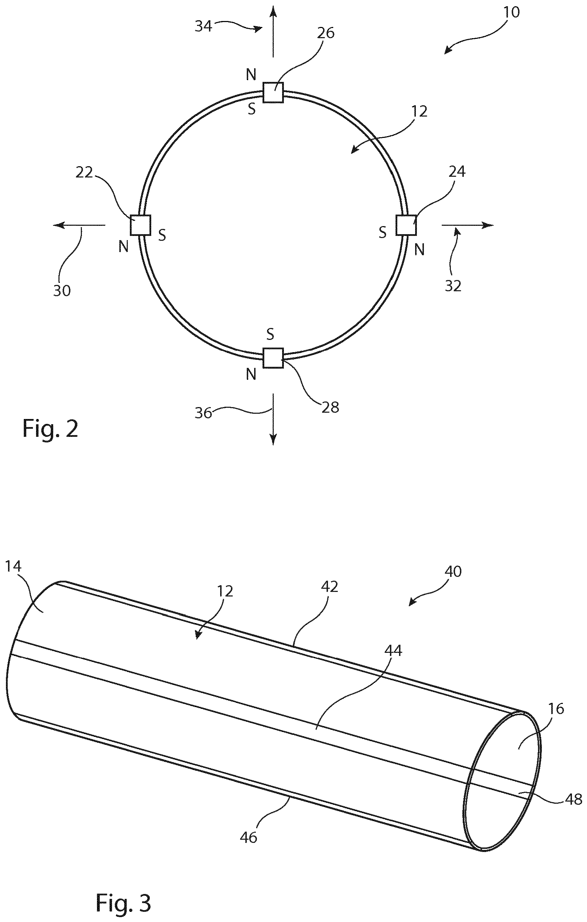

Referring now to Figure 2, this shows a cross-sectional view of the graft of Figure 1, in schematic form. Figure 2 shows arrows 30-36 which depict the magnetic force generated at each of the magnetic elements 22-28 as the result of the opposing magnetic relation with the corresponding magnetic element on the opposing side of the graft tube 12. More specifically, in the described embodiment, the facing south poles of the magnetic elements 22, 24 will produce mutually repulsive magnetic forces in the directions of arrows 30 and 32. Likewise, the opposing magnetic elements 26 and 28 will, by virtue of having their south poles facing one another, also generate mutually repulsive magnetic forces 34 and 36 in opposing directions. These repulsive magnetic forces 30-36 will cause the graft element to be biased in the open configuration, as will be apparent from the schematic diagram of Figure 2.

It will be appreciated that it is preferred that each magnetic element 20 has a directly facing counterpart on the opposing side of the graft tube 12, that is opposing in the radial direction as well in the longitudinal direction along the length of the graft element 12. It is not excluded, however, that the magnetic elements 20 could be slightly offset relative to their opposing counterpart, either in the circumferential direction or in the longitudinal direction or both. However, the offset will produce a lower repulsive force.

Figures 1 and 2 depict an embodiment having two sets of magnetic elements 22, 24 and 26, 28. It is envisaged that some embodiments may have more than two pairs of sets of magnetic elements 20, for instance 4, 6, 8 or more pairs, which again are preferably evenly spaced circumferentially around the tubular graft element 12. Each opposing set of magnetic elements would produce repulsive magnetic forces as with the embodiment of Figure 2, with the addition of further magnetic repulsion between adjacent magnetic elements 20, enhancing the opening of the medical device 10. It is to be understood, though, that in most embodiments it is not necessary to have many magnetic elements 20 on the graft tube 12 as blood flow will tend to keep the graft tube open, the magnetic elements being provided to ensure there is no collapse of the graft tube.

Referring now to Figure 3, this shows another embodiment of implantable medical device 40, which again has a tubular graft element 12 similar to that of the embodiment of Figures 1 and 2 and having similar characteristics. The disk-shaped magnetic elements 22 of the embodiment of Figures 1 and 2 are replaced by strips of magnetic material 42-48, which extend along the length of the graft tube 12, which forms the body portion of the medical device 40. The strips of magnetic material 42-48 extend, in this embodiment, substantially parallel to the longitudinal axis of the tubular graft element 12 and are arranged in opposing pairs, that is with the strips 42, 46 being on opposing sides of the graft tube 12, the strips 44 and 48 extending in like manner. The strips 42-48 have north and south polarities and are arranged such that the same polarity faces internally into the luminal side 16 of the graft tube 12 and the opposite polarity faces the abluminal side 16. In one example, the south pole of each magnetic strip 42-48 extends towards the interior of the graft tube 12 and the north pole faces to the exterior. This arrangement of strips of magnetic material 42-48 provides a very similar functionality to the embodiments of Figures 1 and 2, with a smaller number of magnetic elements being necessary. Again, these strips of magnetic material 42-48 could be provided by printing magnetic material onto the graft tube or by affixing three-dimensional strips to the graft tube.

In the embodiment of Figure 3, the strips 42-48 are continuous along the whole length of the graft element 12, although it is not excluded that these could be discontinuous, that is segmented with or without a gap between adjacent segments of each strip 42-48. Segmenting the strips of magnetic material 42-48 can enhance the longitudinal flexibility of the medical device 40, as will be apparent from the teachings below.

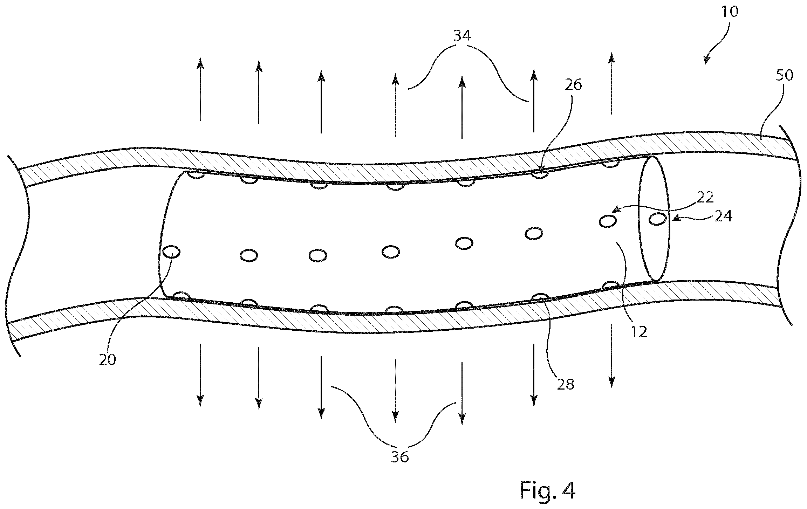

Referring now to Figure 4, this shows the implantable medical device 10 of Figures 1 and 2 disposed within a vessel 50 of a patient. The vessel 50 is shown in cross-section, whereas the implantable medical device 10 is shown in its complete form. Figure 4 also shows the direction of the magnetic repulsive forces 34 and 36 generated by the opposing sets 26, 28 of magnetic elements 20 and the skilled person will appreciate that the equivalent opposing magnetic forces will be generated also at the sets 22 and 24 of magnetic elements and any other opposing sets of magnetic elements. The opposing magnetic forces 34, 36 cause the graft element 12 to be pressed against the vessel wall 50 and thereby ensure that the tubular graft element 12 remains open within the vessel. Furthermore, the repulsive magnetic forces provide a constant opening force to the medical device 10 irrespective of other factors such as vessel movement and the like. As the opening force is magnetic, it is not necessary for the magnetic elements 20 to be particularly large and the graft tube 12 (or other structure of another form of medical device) similarly need not be thick or otherwise voluminous. The medical device 10, therefore, can be very thin, as a result presenting virtually no or otherwise minimal disruption to the flow of fluid within the vessel 50. This can also minimise the amount of foreign material in the body. Moreover, these characteristics also make the device suitable for smaller vessels.

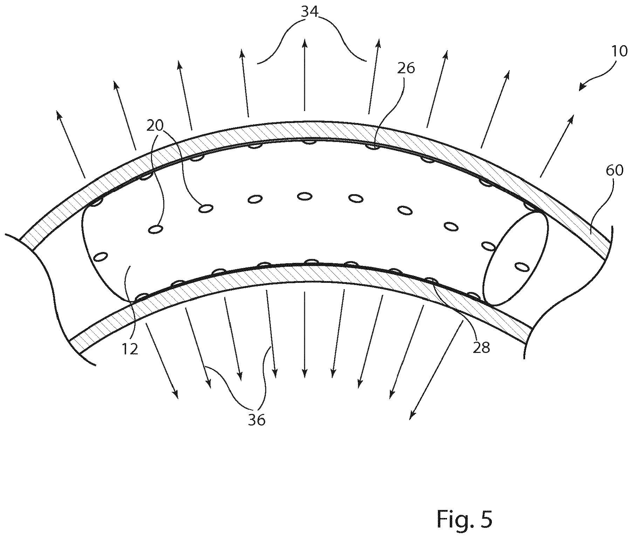

Referring now to Figure 5, this shows the implantable medical device 10 deployed within a curved vessel 60. The medical device 10 is the same as that of Figure 4 and the embodiments of Figures 1 and 2, although it is not excluded that the device 10 could be shaped to correspond with the shape of the vessel 60.

As will be apparent from the force arrows 34, 36 in Figure 5, the opposing magnetic elements will generate the same repulsive magnetic forces but these will be directed in the orthogonal direction relative to the axis vessel 60, thereby ensuring that the graft tubing 12 remains consistently pressed against the vessel wall along the curve, thereby keeping the graft tubing 12 open and in position. These opposing repulsive magnetic forces will therefore be aligned with the curvature of the vessel 60, as depicted in Figure 5. With the embodiment of Figures 1 and 2 as well as with any other embodiment of medical device having segmented or otherwise separate magnetic elements along the length of the graft tubing 12, the structure of device will not exert any straightening force on the vessel 60 of the type which is experienced with conventional implantable medical devices. This makes the medical device disclosed herein suitable for implantation in delicate vessels of a patient, including the cerebral vessels.

In the embodiments of medical device disclosed herein, the magnetic elements could be made from permanent magnets and equally could be made from a paramagnetic material and magnetised after fixation to the body portion of the medical device, for example by means of an electromagnet or a permanent magnetic field. The elements would be magnetised to ensure that the relative polarisation direction of each spot or magnetic strip is identical.

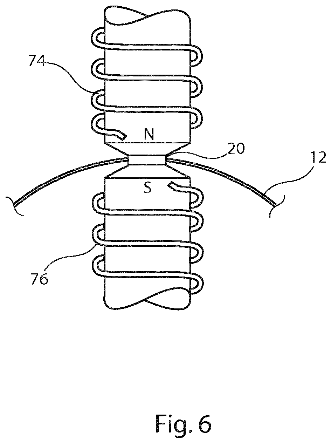

Referring now to Figure 6, this shows an enlarged form, one of the magnetic elements 20 of the embodiment of Figures 1, 2, 4 and 5. In this example, the magnetic element 20 is in the form of a disk or rivet which extends through the body member 12. In this example, the magnetic element 20 is made of a paramagnetic material and magnetisation thereof is achieved by providing electromagnets 72, 74 either side of each magnetic element. The electromagnet 76 can usefully be in the form of a mandrel which is fed into the lumen of the graft. The electromagnet 74 could be a series of electromagnetic elements or a sleeve. The electromagnets 74 and 76 are energised to cause the paramagnetic material of the element 20 to be polarised, in this example to have its south pole on the interior surface of the body member 12 and its north pole extending outwardly of the body portion 12 and therefore of the device 10.

The method of magnetisation of a paramagnetic element 20 may be by means of a permanent magnet or electromagnetically.

In the embodiments described above, there are provided arrays or lines of magnetic elements or strips of magnetic element which extend parallel to the longitudinal axis of the body portion of the medical device 10. In other embodiments, the magnetic elements may extend at an angle to the longitudinal axis and could, for example, extend helically around the body portion or even in zigzag or other curved shape. The exact arrangement and/or shape of the magnetic elements can be varied to give the device 10 different opening characteristics. Equally, some parts of the body portion of the device 10 can be free of magnetic elements in some embodiments.

It is also envisaged that the magnetic elements could extend circumferentially around the body portion, in one or more annular bands. Each strip could be simply annular or undulating or of zigzag form.

It is envisaged that the medical device 10 could be deployed in a patient's vessel over an inflatable delivery balloon. Such a balloon can ensure that the body member 12 of the device is expanded properly against the vessel wall, with the magnetic elements 20 then ensuring that the device 10 expands as desired.

The magnetic elements could be made of: NdFeB (neodymium), FeCrCo, SMCo or PtCo.

It is envisaged that the magnetic elements could have a thickness of 0.10 millimetres or more.

It is preferred that the magnetic elements are formed from a biodegradable or bioabsorbable material, likewise with the other elements of the device.

It is not necessary for the medical device to have a graft element. In some embodiments the magnetic elements could be fitted, for example, to a stent or other open structure, for example allowing the stent to have a smaller and weaker mechanical structure than conventional stents.

## Claims

1. A medical device (10) including an expandable body member (12) having a cylindrical tubular shape, the expandable body member including at least first and second sides with opposing internal surfaces (16), at least first and second magnetic elements (20) each located at a respective one of said opposing internal surfaces so as to be disposed in opposing relation to one another, wherein each magnetic element has a north pole and a south pole, **characterized in that** the magnetic elements are disposed so as to have the same pole facing inwardly towards the central axis of the device and the opposing pole facing outwardly thereby to generate a repulsive force between one another.

2. A device (10) according to claim 1, wherein the device is an implantable medical device.

3. A device (10) as claimed in claim 1 or 2, wherein the body member (12) is formed of knitted, woven, braided or sheet material.

4. A device (10) as claimed in claim 1, 2 or 3, wherein the device is or includes a graft (12), the graft including said body member.

5. A device (10) as claimed in any preceding claim, including a stent at either end of the body member (12).

6. A device (10) as claimed in any preceding claim, wherein the magnetic elements (20) are disks or rivets.

7. A device (10) as claimed in any preceding claim, including at least first and second lines (22, 24, 26, 28) of magnetic elements (20) along the body portion of the device, optionally including at least two pairs of lines of magnetic elements, the lines in each pair being disposed on opposing internal surfaces (16) of the body member (12).

8. A device (10) as claimed in any of claims 1 to 5, wherein the magnetic elements (20) are strips of magnetic material, optionally including at least two pairs of strips (42, 44, 46, 48) of magnetic material, the strips in each pair being disposed on opposing internal surfaces (16) of the body member (12).

9. A device (10) as claimed in claim 7 or 8, wherein the body member (12) of the device has an axial dimension and said lines (22, 24, 26, 28) of magnetic elements (20) or strips (42, 44, 46, 48) of magnetic material extend parallel to the axial dimension of the body member.

10. A device (10) as claimed in claim 7 or 8, wherein the body member (12) of the device has an axial dimension and said lines (22, 24, 26, 28) of magnetic elements (20) or strips (42, 44, 46, 48) of magnetic material extend at an angle to the axial dimension of the body member.

11. A device (10) as claimed in claim 10, wherein the lines (22, 24, 26, 28) of magnetic elements (20) or strips (42, 44, 46, 48) of magnetic material extend helically around the body member (12) or wherein the lines of magnetic elements or strips of magnetic material extend in a zigzag manner along the body member.

12. A device (10) as claimed in any of claims 9 to 11, wherein the strips (42, 44, 46, 48) of magnetic material are discontinuous along the body member (12).

13. A device (10) as claimed in any preceding claim, wherein the magnetic elements (20) are painted on, printed on or attached to the body member (12) of the device.

14. A device (10) as claimed in any preceding claim, wherein the magnetic elements (20) are formed of paramagnetic material.

15. A device (10) as claimed in any preceding claim, wherein the magnetic elements (20) are formed of biodegradable or bioabsorbable material.

## Patentansprüche

1. Medizinische Vorrichtung (10), die ein expandierbares Körperglied (12) mit zylindrischer Röhrenform einschließt, wobei das expandierbare Körperglied mindestens erste und zweite Seiten mit gegenüber liegenden Innenflächen (16) einschließt, wobei mindestens erste und zweite magnetische Elemente (20) sich jeweils an einer der jeweiligen gegenüber liegenden Innenflächen befinden, um so in einander gegenüber liegender Beziehung angeordnet zu sein, wobei jedes Magnetelement einen Nordpol und einen Südpol aufweist, **dadurch gekennzeichnet, dass**
die Magnetelemente so angeordnet sind, dass der gleiche Pol einwärts in Richtung der Zentralachse der Vorrichtung weist und der gegenüber liegende Pol dadurch auswärts weist, um eine abstoßende Kraft zwischen den Elementen zu generieren.

2. Vorrichtung (10) nach Anspruch 1, wobei die Vorrichtung eine implantierbare medizinische Vorrichtung ist.

3. Vorrichtung (10) nach Anspruch 1 oder 2, wobei das Körperglied (12) aus gestricktem, gewebtem, geflochtenem oder Lagenmaterial gebildet ist.

4. Vorrichtung (10) nach Anspruch 1, 2 oder 3, wobei die Vorrichtung ein Transplantat (12) ist oder einschließt, wobei das Transplantat das Körperglied einschließt.

5. Vorrichtung (10) nach einem der vorhergehenden Ansprüche, einschließlich eines Stents an jedem Ende des Körperglieds (12).

6. Vorrichtung (10) nach einem der vorhergehenden Ansprüche, wobei die Magnetelemente (20) Scheiben oder Nieten sind.

7. Vorrichtung (10) nach einem der vorhergehenden Ansprüche, die mindestens erste und zweite Linien (22, 24, 26, 28) von Magnetelementen (20) entlang des Körperabschnitts der Vorrichtung einschließt, wobei gegebenenfalls mindestens zwei Paare von Linien von Magnetelementen eingeschlossen sind, wobei die Linien in jedem Paar auf gegenüber liegenden Innenflächen (16) des Körperglieds (12) angeordnet sind.

8. Vorrichtung (10) nach einem der Ansprüche 1 bis 5, wobei die Magnetelemente (20) Streifen aus magnetischem Material sind, wobei gegebenenfalls mindestens zwei Paare von Streifen (42, 44, 46, 48) aus magnetischem Material eingeschlossen sind, wobei die Streifen in jedem Paar auf gegenüber liegenden Innenflächen (16) des Körperglieds (12) angeordnet sind.

9. Vorrichtung (10) nach Anspruch 7 oder 8, wobei das Körperglied (12) der Vorrichtung eine Axialabmessung aufweist und die Linien (22, 24, 26, 28) der Magnetelemente (20) oder Streifen (42, 44, 46, 48) des magnetischen Materials sich parallel zu der Axialabmessung des Körperglieds erstrecken.

10. Vorrichtung (10) nach Anspruch 7 oder 8, wobei das Körperglied (12) der Vorrichtung eine Axialabmessung aufweist und die Linien (22, 24, 26, 28) der Magnetelemente (20) oder Streifen (42, 44, 46, 48) des magnetischen Materials sich in einem Winkel zu der Axialabmessung des Körperglieds erstrecken.

11. Vorrichtung (10) nach Anspruch 10, wobei die Linien (22, 24, 26, 28) der Magnetelemente (20) oder Streifen (42, 44, 46, 48) des magnetischen Materials sich spiralig um das Körperglied (12) erstrecken, oder wobei die Linien der Magnetelemente oder Streifen des magnetischen Materials sich zickzackartig entlang des Körperglieds erstrecken.

12. Vorrichtung (10) nach einem der Ansprüche 9 bis 11, wobei die Streifen (42, 44, 46, 48) des magnetischen Materials entlang des Körperglieds (12) diskontinuierlich sind.

13. Vorrichtung (10) nach einem der vorhergehenden Ansprüche, wobei die Magnetelemente (20) auf das Körperglied (12) der Vorrichtung aufgemalt, auf dieses aufgedruckt oder an diesem befestigt sind.

14. Vorrichtung (10) nach einem der vorhergehenden Ansprüche, wobei die Magnetelemente (20) aus paramagnetischem Material gebildet sind.

15. Vorrichtung (10) nach einem der vorhergehenden Ansprüche, wobei die Magnetelemente (20) aus bioabbaubarem oder bioabsorbierbarem Material gebildet sind.

## Revendications

1. Dispositif médical (10) comprenant un élément de corps extensible (12) ayant une forme tubulaire cylindrique, l'élément de corps extensible comprenant au moins des premier et second côtés avec des surfaces internes opposées (16), au moins des premier et second éléments magnétiques (20) situés chacun au niveau d'une surface respective desdites surfaces internes opposées de manière à être disposés en relation opposée l'un à l'autre, chaque élément magnétique ayant un pôle nord et un pôle sud, **caractérisé en ce que** les éléments magnétiques sont disposés de manière à avoir le même pôle orienté vers l'intérieur vers l'axe central du dispositif et le pôle opposé orienté vers l'extérieur afin de générer une force de répulsion entre eux.

2. Dispositif (10) selon la revendication 1, le dispositif étant un dispositif médical implantable.

3. Dispositif (10) selon la revendication 1 ou 2, l'élément de corps (12) étant formé d'un matériau tricoté, tissé, tressé ou en feuille.

4. Dispositif (10) selon la revendication 1, 2 ou 3, le dispositif étant ou comprenant une greffe (12), la greffe comprenant ledit élément de corps.

5. Dispositif (10) selon l'une quelconque des revendications précédentes, comprenant un stent à chaque extrémité de l'élément de corps (12).

6. Dispositif (10) selon l'une quelconque des revendications précédentes, les éléments magnétiques (20) étant des disques ou des rivets.

7. Dispositif (10) selon l'une quelconque des revendications précédentes, comprenant au moins des première et deuxième lignes (22, 24, 26, 28) d'éléments magnétiques (20) le long de la partie de corps du dispositif, comprenant éventuellement au moins deux paires de lignes d'éléments magnétiques, les lignes de chaque paire étant disposées sur des surfaces internes opposées (16) de l'élément de corps (12).

8. Dispositif (10) selon l'une quelconque des revendications 1 à 5, les éléments magnétiques (20) étant des bandes de matériau magnétique, comprenant éventuellement au moins deux paires de bandes (42, 44, 46, 48) de matériau magnétique, les bandes de chaque paire étant disposées sur des surfaces internes opposées (16) de l'élément de corps (12).

9. Dispositif (10) selon la revendication 7 ou 8, l'élément de corps (12) du dispositif ayant une dimension axiale et lesdites lignes (22, 24, 26, 28) d'éléments magnétiques (20) ou de bandes (42, 44, 46, 48) de matériau magnétique s'étendant parallèlement à la dimension axiale de l'élément de corps.

10. Dispositif (10) selon la revendication 7 ou 8, l'élément de corps (12) du dispositif ayant une dimension axiale et lesdites lignes (22, 24, 26, 28) d'éléments magnétiques (20) ou de bandes (42, 44, 46, 48) de matériau magnétique s'étendant selon un angle par rapport à la dimension axiale de l'élément de corps.

11. Dispositif (10) selon la revendication 10, les lignes (22, 24, 26, 28) d'éléments magnétiques (20) ou de bandes (42, 44, 46, 48) de matériau magnétique s'étendant en hélice autour de l'élément de corps (12) ou les lignes d'éléments magnétiques ou de bandes de matériau magnétique s'étendant en zigzag le long de l'élément de corps.

12. Dispositif (10) selon l'une quelconque des revendications 9 à 11, les bandes (42, 44, 46, 48) de matériau magnétique étant discontinues le long de l'élément de corps (12).

13. Dispositif (10) selon l'une quelconque des revendications précédentes, les éléments magnétiques (20) étant peints sur, imprimés sur ou fixés à l'élément de corps (12) du dispositif.

14. Dispositif (10) selon l'une quelconque des revendications précédentes, les éléments magnétiques (20) étant formés d'un matériau paramagnétique.

15. Dispositif (10) selon l'une quelconque des revendications précédentes, les éléments magnétiques (20) étant formés d'un matériau biodégradable ou bioabsorbable.
